# FASCICULE DE BREVET EUROPEEN

(11) **EP 2 582 734 B1**
(45) Date de publication et mention de la délivrance du brevet: **17.12.2014**
(21) Numéro de dépôt: 11727233.6
(22) Date de dépôt: 19.05.2011
(51) Int. Cl.: C08F 220/68, C08F 2/32, A61K 47/32, A61K 8/81, A61K 8/06, A61K 8/90, A61Q 19/00, C08F 220/18, C08F 220/34, C08F 220/58

(54) **NOUVEAUX POLYMÈRES ÉPAISSISSANTS DE PHASES GRASSES**
NEUE VERDICKUNGSPOLYMERE FÜR ÖLPHASEN
NOVEL THICKENING POLYMERS FOR OIL PHASES

(30) Priorité: 15.06.2010 FR 1054705
(43) Date de publication de la demande: 24.04.2013
(73) Titulaire: Societe D Exploitation De Produits Pour Les Industries Chimiques Seppic, 75007 Paris (FR)
(72) Inventeur: BRAUN, Olivier, F-81100 Castres (FR); OLLAGNIER, Jean-Noël, F-81100 Castres (FR)
(74) Mandataire: Conan, Philippe Claude
(86) Numéro de dépôt international: PCT/FR2011/051136
(87) Numéro de publication internationale: WO 2011/157914

(56) Documents cités:
- EP-A2- 2 011 481
- WO-A1-01/35922
- FR-A1- 2 786 493
- FR-A1- 2 873 126
- US-A- 5 736 125
- US-A1- 2003 021 756

## Description

La présente demande de brevet concerne de nouveaux composés épaississant les phases huile, leur procédé de préparation et leur application en tant qu'épaississants et/ou émulsionnants pour des produits de soins de la peau, des cheveux et du cuir chevelu notamment les émulsions cosmétiques, dermocosmétiques, dermopharmaceutiques ou pharmaceutiques eau-dans huile.

Des polymères épaississants synthétiques, se présentant sous forme de latex inverses, sont décrits comme pouvant être utilisés dans la fabrication de compositions topiques, dans les demandes de brevet européen publiées sous les numéros EP 0 716 594, EP 1 047 716, EP 1 056 805 et EP 0 503 853. Cependant, la plupart de ces épaississants ne sont pas capables d'épaissir les phases huile.

La demande de brevet européen publiée sous le numéro EP 0 406 042 divulgue des compositions cosmétiques sous forme d'émulsions eau dans huile contenant comme agent épaississant un polymère ayant une faible proportion de motifs à groupement ioniques tel que par exemple le copolymère de N-dodécyl acrylamide et d'acide acrylamido-2 méthyl-2 propanesulfonique (AMPS) dans une proportion pondérale 96,5/3,5 ou le copolymère de N-tertiobutyl acrylamide et d'acide acrylamido-2 méthyl-2 propanesulfonique dans une proportion pondérale 97,9/2,1. De tels copolymères sont d'un usage délicat dans l'industrie cosmétique, car ils doivent être neutralisés avant utilisation, et que leur solubilisation dans les huiles nécessite souvent l'emploi d'un co-solvant. De plus leur aptitude à épaissir les huiles est faible.

C'est la raison pour laquelle les seuls composés connus aujourd'hui possédant cette propriété, sont les copolymères commercialisés sous le nom INTELIMER qui sont copolymères hydrophobes ayant de longues chaînes alkyle pendantes qui cristallisent à froid pour former des clusters provoquant l'épaississement du milieu. De tels polymères du type copolymères d'acrylate d'alkyle et d'acide (méth)acrylique sont décrits dans les brevets américains publiés sous les numéros US 7,101,928 B1 et US 5,736,125. Le brevet américain US 5,736,125 divulgue notamment des copolymères acrylate de stéaryle/acide méthacrylique, dans un rapport massique hydrophobe/hydrophile compris entre 80/20 et 98/2. Néanmoins l'utilisation de ces produits n'est pas simple car il faut dissoudre le polymère à chaud dans l'huile puis procéder à un refroidissement pour provoquer la cristallisation des chaines. De plus ils sont par nature sensibles à la température et les compositions épaissies avec ce type de polymère sont délicates à commercialiser dans les pays chauds.

C'est pourquoi la demanderesse a cherché à développer de nouveaux polymères capables d'épaissir les phases huiles et organiques qui n'aient pas les inconvénients exposés ci-dessus.

Elle a trouvé que des polymères de type ionomères caractérisés par la présence de groupes ioniques sur un squelette hydrophobe constituaient une classe d'épaississants d'huile intéressante. L'invention a donc pour objet des copolymères de type ionomères en multiblocs ainsi qu'un procédé pour leur préparation qui repose sur la polymérisation d'un monomère hydrophobe en solution contenant une faible proportion de monomères hydrophiles solubilisés au sein de micelles de tensioactifs.

Ce copolymère ionomère multibloc est caractérisé en ce qu'il comporte pour 100% molaire :
a) de 90% molaire à 99,9% molaire d'unités monomériques hydrophobes (A) issues d'un composé de formule (I) : 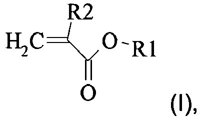 dans laquelle R1 représente un radical alkyle, linéaire ou ramifié, comportant de 12 à 22 atomes de carbone et R2 représente un atome d'hydrogène ou un radical méthyle ; et soit
b) de 0,1% molaire à 10% molaire d'unités monomériques (B) issue de l'acide 2-méthyl 2-[(1-oxo 2-propènyl) amino] 1-propanesulfonique (dénommé aussi acide 2-acrylamido 2-méthyl propanesulfonique) partiellement ou totalement salifiée, soit
c) de 0,1% molaire à 10% molaire d'unités monomériques (C) issues d'au moins un monomère cationique, soit
d) de 0,1% molaire à 10% molaire d'à la fois, de dites unités monomériques (B) et de dites unités monomériques (C).

Dans la formule (I) telle que définie précédemment, lorsque le radical R1 représente un radical alkyle linéaire comprenant de 12 à 22 atomes de carbone, il s'agit plus particulièrement d'un radical choisi parmi les radicaux dodécyle, tétradécyle, hexadécyle, octadécyle, eicosyle ou docosyle.

Dans la formule (I) telle que définie précédemment, lorsque le radical R1 représente un radical alkyle ramifié comprenant de 12 à 22 atomes de carbone, il peut s'agir du radical de formule :

CH(CₙH₂ₙ₊₁)(CₘH₂ₘ₊₁)-CH₂-

dans lequel m est un nombre entier égal compris entre 1 et 4, n est un nombre entier compris entre 9 et 16 ; ou du radical de formule :

CH(CₚH₂ₚ₊₁)₂ -CH₂-

dans lequel p est un nombre entier égal compris entre 5 et 10 ;

Selon un aspect particulier de la présente invention, dans la formule (I) telle que définie précédemment, le radical R1 représente un radical alkyle linéaire choisi parmi les radicaux tétradécyle, hexadécyle, octadécyle, écosyle ou docosanyle, et tout particulièrement choisi parmi les radicaux, hexadécyle, octadécyle ou écosyle.

Dans la définition de la présente invention, le terme salifié indique qu'il s'agit de sels de métaux alcalins tels que les sels de sodium ou de potassium, les sels de bases azotées comme le sel d'ammonium, le sel de lysine ou le sel de monoéthanolamine (HO-CH₂-CH₂-NH₄⁺). Selon un aspect particulier de la présente invention, par salifié, on signifie désigne que la fonction acide est salifiée sous forme de sel de sodium.

Dans la définition de l'invention, par monomère cationique dont est issue ladite unité monomérique (C), on désigne plus particulièrement un dérivé d'ammonium quaternaire choisis parmi les sels de N,N,N-triméthyl 2-[2-méthyl (1-oxo-2-propènyl) amino] propanammonium (MAPTAC), les sels de N,N,N-triméthyl 2-[(1-oxo-2-propènyl) oxy] éthanammonium (ADQUAT), les sels de N,N,N-triméthyl 3-[(1-oxo-2-propènyl) oxy] propanammonium, les sels de N,N,N-triméthyl 3-[(1-oxo-2-propènyl) amino] propanammonium (APTAC), les sels de N,N,N-triméthyl 2-[(2-méthyl 1-oxo-2-propènyl) oxy] éthanammonium (MADQUAT) ou les sels de diallyl diméthyl ammonium (DADMAC).

Par sel on désigne les anions d'acides forts et plus particulièrement, les halogénures tels que les chlorures ou les bromures.

Selon un mode particulier, l'invention a pour objet un copolymère ionomère multibloc tel que défini précédemment, comportant pour 100% molaire :
a) de 95% molaire à 99% molaire d'unités monomériques hydrophobes (A) ; et soit
b) de 1% molaire à 5% molaire d'unités monomériques (B) ; soit
c) de 1% molaire à 5% molaire d'unités monomériques (C) ; soit
d) de 1% molaire à 5% molaire d'à la fois de dites unités monomériques (B) et de dites unités monomériques (C).

Selon un autre aspect particulier de la présente invention, dans le copolymère ionomère multibloc tel que défini précédemment, les ratios molaires (A)/(B), (A)/(C) ou (A)/[(B)+(C)] sont inférieurs ou égaux à 99/1 et plus particulièrement inférieurs ou égaux à 98/2.

L'invention a plus particulièrement pour objet, soit un copolymère ionomère multiblocs de méthacrylate de stéaryle et de 2-méthyl 2-[(1-oxo 2-propènyl) amino] 1-propanesulfonate de sodium, pour lequel le ratio molaire (A)/(B) est supérieur ou égal à 95/5 et inférieur ou égal à 98/2, soit un copolymère ionomère multiblocs de méthacrylate de stéaryle et de chlorure de N,N,N-triméthyl 2-[(2-méthyl 1-oxo-2-propènyl) oxy] éthanommonium, pour lequel le ratio molaire (A)/(C) est supérieur ou égal à 95/5 et inférieur ou égal à 98/2, soit un copolymère ionomère multiblocs de méthacrylate de stéaryle, de 2-méthyl 2-[(1-oxo 2-propènyl) amino] 1-propanesulfonate de sodium et de chlorure de N,N,N-triméthyl 2-[(2-méthyl 1-oxo-2-propènyl) oxy] éthanommonium, pour lequel le ratio molaire (A)/(B)/(C) est compris entre 92/4/4 et 98/1/1.

L'invention a aussi pour objet un procédé de préparation du copolymère ionomère multibloc tel que défini précédemment, comprenant :
- Une étape a) de préparation d'une phase organique par mélange d'au moins une huile apolaire avec le monomère de formule (I) et un système tensioactif (T), susceptible de former des micelles inverses ;
- Le cas échéant, une étape b) d'addition dans la phase organique préparée à l'étape a), d'une solution aqueuse comprenant ledit monomère comportant la fonction acide sulfonique ;
- Le cas échéant, une étape b') d'addition dans le mélange préparé à l'étape a) ou dans le mélange préparé à l'étape b), d'une solution aqueuse comprenant ledit monomère cationique ;
- Une étape c) de polymérisation amorcée par introduction dans le mélange résultant de l'étape b) ou de l'étape b'), d'un amorceur thermique ; et
- Une étape d) d'élimination de l'huile et si désiré, dudit système tensioactif.

Par huile apolaire, on désigne dans le procédé tel que défini précédemment, une huile ou un mélange d'huiles apolaires choisies parmi les paraffines les isoparaffines ou les cycloparaffines présentant à température ambiante, une densité entre 0,7 et 0,9 et un point d'ébullition supérieur à environ 150°C, telle que par exemple :
- L'ISOPAR™ H, l'ISOPAR™ G ou l'ISOPAR™ M,
- Le MARCOL™ 52, une huile commerciale répondant à la définition des huiles de vaseline du Codex français et qui est une huile blanche minérale conforme aux réglementations FDA 21 CFR 172.878 et CFR 178.3620 (a) elle est inscrite à la Pharmacopée des USA, US XXIII (1995) et à la Pharmacopée européenne (1993) ;
- L'isohexadécane, identifié dans Chemical-Abstract par le numéro RN = 4390-04-9 qui est un mélange d'isoparaffines en C12, C16, C20 contenant au moins 97% d'isoparaffines en C16 parmi lesquelles le constituant principal est le 2, 2, 4, 4, 6, 8, 8 - heptaméthyl nonane (RN = 4390-04-9),
- L'isododécane.

Par système tensioactif (T), susceptible de former des micelles inverses, on désigne dans le procédé tel que défini précédemment, un tensioactif ou un mélange d'agents tensioactifs. Comme exemple de tensioactif approprié à la mise en oeuvre du procédé tel que défini précédemment, il y a par exemple le sulfosuccinate de dioctyle, commercialisé par la demanderesse sous le nom TRITON™ GR7. On peut également citer les esters de sorbitan ayant particulièrement un HLB entre 3,5 et 8 tel que le mono-oléate de sorbitan ou l'isostéarate de sorbitan, ou les mélanges de ces tensioactifs.

Selon un mode particulier de cette étape a), on ajoute d'abord le composé de formule (I) telle que définie précédemment, puis on ajoute ledit système tensioactif (T), pour former une solution d'huile comprenant pour 100% massique, entre 20% massique et 70% massique et plus particulièrement entre 35% massique et 55% massique dudit monomère de formule (I), et entre 5% et 20 % et plus particulièrement entre 10% à 15% massique dudit système tensioactif (T).

L'étape b) du procédé tel que défini précédemment, est réalisée sous agitation jusqu'à obtenir une dispersion homogène, comportant l'ensemble des monomères dans les proportions molaires souhaitées.

Selon un mode particulier de cette étape b), La phase aqueuse comprend pour 100% massique, entre 10% massique et 75% massique et plus particulièrement entre 15% et 45% massique de monomères hydrophiles.

L'étape c) du procédé tel que défini précédemment, est typiquement réalisée à une température d'environ 80°C, et laissée se dérouler jusqu'à polymérisation complète.

Par amorceur thermique on désigne dans l'étape c) du procédé tel que défini précédemment, amorceur thermique, notamment l'azo bis(isobutyronitrile) (AIBN) ou le peroxyde de lauroyle.
- L'étape d) d'élimination de l'huile et si désiré dudit système tensioactif est généralement réalisée par précipitation préalable du copolymère formé à l'issue de l'étape c) avec un solvant de précipitation approprié tel que par exemple l'acétone, puis filtration du précipité. En utilisant différents solvants et en effectuant des étapes de rinçage avec chacun des dits différents solvants, il est possible d'éliminer l'huile et le tensioactif.

L'invention a aussi pour objet une variante du procédé tel que défini ci-dessus, dans lequel l'étape d) est limitée à l'élimination de l'huile pour former un mélange dudit copolymère ionomère multiblocs et dudit système tensioactif susceptible de former des micelles inverses. Selon cette variante, l'étape d'élimination consiste de préférence en une étape d'atomisation pour éliminer uniquement l'huile.

L'invention a aussi pour objet une composition (F) comprenant pour 100% massique :
de 60% à 90% en massique, dudit copolymère ionomère multibloc telque défini précédemment, et
de 10% à 40% massique dudit système tensioactif susceptible de former des micelles inverses, et plus particulièrement la composition telle que définie ci-dessus, directement obtenue par la variante du procédé tel que définie précédemment.

L'invention a aussi pour objet l'utilisation du copolymère ionomère multibloc ou de la composition (F) tels que définis précédemment, pour préparer une composition topique cosmétique, dermopharmaceutique ou pharmaceutique et plus particulièrement l'utilisation telle que définie ci-dessus, dans laquelle la composition topique cosmétique, dermopharmaceutique ou pharmaceutique est une émulsion de type eau dans huile.

Une composition topique selon l'invention, destinée à être appliquée sur la peau, sur le cheveu, sur le cuir chevelu ou sur les muqueuses de l'homme ou de l'animal, peut consister en une émulsion topique comprenant au moins une phase aqueuse et au moins une phase huile. Cette émulsion topique est plus particulièrement du type eau dans huile. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau, du cuir chevelu et des muqueuses. Dans ce dernier cas, la composition topique comporte alors un principe actif qui peut par exemple consister en un agent anti-inflammatoire, un agent myorelaxant, un agent antifongique ou un agent antibactérien.

Lorsque la composition topique est utilisée en tant que composition cosmétique destinée à être appliquée sur la peau, sur le cuir chevelu ou les muqueuses, elle peut ou non comporter un principe actif, par exemple un agent hydratant, un agent bronzant, un filtre solaire, un agent antirides, un agent à visée amincissante, un agent anti -radicalaire, un agent anti-acnéique ou un agent antifongique.

La composition topique peut en outre comporter des composés classiquement compris dans ce type de compositions, par exemple des parfums, des conservateurs, des antioxydants, des colorants, des émollients ou des tensioactifs.

L'invention a enfin pour objet un procédé pour modifier la rhéologie d'une composition topique cosmétique, dermopharmaceutique ou pharmaceutique comprenant une phase grasse, caractérisé en ce que l'on introduit dans ladite phase grasse une quantité efficace copolymère ionomère multibloc ou de la composition (F) tels que définis précédemment.

Une composition topique selon l'invention et plus particulièrement une émulsion eau dans huile selon invention comporte habituellement pour 100% de sa masse totale entre 0,1% massique et 10% massique et plus particulièrement entre 1% massique et 5 % massique du copolymère ionomère multiblocs tel que défini ci-dessus.

Les exemples qui suivent ont pour but d'illustrer la présente invention.

### Exemple 1 : Préparation du copolymère ionomère multiblocs (AMPS, sel de Na) / méthacrylate de stéaryle (MAS) (2 / 98) (Composé 1)

a) On introduit d'abord 51,564 g d'ISOPAR™ H dans un réacteur. On verse ensuite progressivement et sous agitation en maintenant la température inférieure à 25 °C, 34,376 g de méthacrylate de stéaryle puis 10,91 g de sulfosuccinate de dioctyle.
b) On prépare une solution aqueuse à 15% massique de 2-méthyl 2-[(1-oxo 2-propènyl) amino] 1-propanesulfonate de sodium.
c) On verse progressivement 3,15 g de la solution aqueuse préparée à l'étape b), dans le réacteur contenant la phase huileuse préparée à l'étape a). Après dégazage sous argon pendant environ une demi-heure, la température est portée à 80°C puis la polymérisation est initiée par ajout de 0,5% molaire d'azo-bis(isobutyronitrile). On laisse la polymérisation se dérouler pendant 7 heures et l'on obtient le copolymère attendu en dispersion dans l'huile.
d) On fait précipiter le copolymère en versant lentement, la dispersion obtenue à l'étape c) dans environ 150 ml d'acétone puis on filtre le précipité et on le sèche pour obtenir une poudre du copolymère attendu.

### Exemple 2 : Préparation du copolymère ionomère multiblocs (AMPS, sel de Na) / méthacrylate de stéaryle (MAS) (4/96) (Composé 2)

On opère de la manière qu'à l'exemple 1 précédent mais en utilisant les quantités de de solvants et de réactants suivants :
Isopar™ H : 42,71 g
Méthacrylate de stéaryle : 43,23 g
Sulfosuccinate de dioctyle : 10,91 g
2-méthyl 2-[(1-oxo 2-propènyl) amino] 1-propanesulfonate de sodium : 3,15 g d'une solution aqueuse à 34,73% massique du sel de sodium.

### Exemple 3 : Préparation du copolymère ionomère multiblocs (MADQUAT) / méthacrylate de stéaryle (MAS) / (4 / 96) (Composé 3)

On opère de la même manière qu'à l'exemple 2 mais on remplace la solution aqueuse de 2-méthyl 2-[(1-oxo 2-propènyl) amino] 1-propanesulfonate de sodium par 3,15 g d'une solution aqueuse à 75% massique de chlorure de N,N,N-triméthyl 2-[(2-méthyl 1-oxo-2-propènyl) oxy] éthanammonium.

### Exemple 4 : Préparation du copolymère ionomère zwitterionique (MAS / AMPS / MADQUAT) 96/2/2 (Composé 4)

a) On introduit d'abord 130 g d'Isopar™ H dans un réacteur. On verse ensuite progressivement et sous agitation en maintenant la température inférieure à 25°C, 128 g de méthacrylate de stéaryle puis 31,5 g de sulfosuccinate de dioctyle.
b) On prépare une solution aqueuse à 75% massique de chlorure de N,N,N-triméthyl 2-[(2-méthyl 1-oxo-2-propènyl)oxy] éthanammonium.
c) On verse progressivement 3,29 g d'une solution aqueuse commerciale à 55% massique de 2-méthyl 2-[(1-oxo 2-propènyl) amino] 1-propanesulfonate de sodium, puis 2,17 g de la solution aqueuse de monomère cationique préparée à l'étape b), dans le réacteur contenant la phase huileuse préparée à l'étape a). Après dégazage sous argon pendant environ une demi-heure, la température est portée à 80°C puis la polymérisation est initiée par ajout de 0,5% molaire d'azo-bis(isobutyronitrile). On laisse la polymérisation se dérouler pendant 7 heures et l'on obtient le copolymère attendu en dispersion dans l'huile.
d) On fait précipiter le copolymère en versant lentement, la dispersion obtenue à l'étape c) dans environ 150 ml d'acétone puis on filtre le précipité et on le sèche pour obtenir une poudre su copolymère attendu.

### Exemple 5 : Préparation du copolymère ionomère zwitterionique (MAS/AMPS/MADQUAT) 94/3/3 (Composé 5)

On opère de la même manière qu'à l'exemple 4 précédent, mais en utilisant les quantités de solvants et de réactants suivants :
Isopar™ H : 132,67 g
Méthacrylate de stéaryle : 125,33 g
Sulfosuccinate de dioctyle : 31,5 g
2-méthyl 2-[(1-oxo 2-propènyl) amino] 1-propanesulfonate de sodium : 4,94 g d'une solution aqueuse commerciale à 55% massique de 2-méthyl 2-[(1-oxo 2-propènyl) amino] 1-propanesulfonate de sodium ;
Chlorure de N,N,N-triméthyl 2-[(2-méthyl 1-oxo-2-propènyl) oxy] éthanammonium : 3,26 g d'une solution aqueuse à 75% massique de chlorure de N,N,N-triméthyl 2-[(2-méthyl 1-oxo-2-propènyl) oxy] éthanammonium.

### Exemple A : Préparation du copolymère ionomère multiblocs Acrylate de sodium / méthacrylate de stéaryle (MAS) (4 / 96) (Composé A selon l'état de la technique)

On opère de la manière qu'à l'exemple 1 précédent mais en utilisant les quantités de solvants et de réactants suivants :
Isopar™ H : 42,71 g
Méthacrylate de stéaryle : 43,23 g
Sulfosuccinate de dioctyle : 10,91 g
Acrylate de sodium : 3 g d'une solution aqueuse à 15% massique d'acrylate de sodium

### B) Evaluation des pouvoirs épaississants des copolymères des exemples 2, 3 et 4 ainsi que de l'exemple A selon l'état de la technique

On mélange 0,5 g de chacun des composés 2, 3, 4 et A dans 9,5 g d'huile à tester. Chacun des échantillons de phase grasse est ensuite placé 30 secondes au bain marie à 80°C puis maintenu sous agitation à 500 tours par minute pendant 3 heures environ, jusqu'à la dissolution complète du polymère. La solubilité est évaluée au cours du temps. Les résultats sont consignés dans le tableau ci-dessous, accompagnés d'une évaluation de la consistance des gels obtenus.

| Composé 2 | | | | |
|---|---|---|---|---|
| | Isohexadécane | Triglycéride C8C10 | Huile de jojoba | Marcol™52 |
| 1h d'agitation | Soluble | Solubilité partielle | Soluble | Soluble |
| 2h d'agitation | Soluble | Solubilité partielle | Soluble | Soluble |
| 3h d'agitation | Soluble | Soluble | Soluble | Soluble |
| Consistance du gel final | ** | * | ** | ** |

| Composé 3 | | | | |
|---|---|---|---|---|
| | Isohexadécane | Triglycéride C8C10 | Huile de jojoba | Marcol™ 52 |
| 1h d'agitation | Soluble | Solubilité partielle | Soluble | Soluble |
| 2h d'agitation | Soluble | Soluble | Soluble | Soluble |
| 3h d'agitation | Soluble | Soluble | Soluble | Soluble |
| Consistance du gel final | ** | * | ** | ** |
| | | | | |

| Composé 4 | | | | |
|---|---|---|---|---|
| | Isohexadécane | Triglycéride C8C10 | Huile de jojoba | Marcol™52 |
| 1h d'agitation | Soluble | Solubilité partielle | Solubilité partielle | Soluble |
| 2h d'agitation | Soluble | Solubilité partielle | Soluble | Soluble |
| 3h d'agitation | Soluble | Soluble | Soluble | Soluble |
| Consistance du gel final | *** | * | *** | *** |

| | | | | |
|---|---|---|---|---|
| Echelle de viscosité : * : Peu visqueux, ** : Visqueux, *** : Plus visqueux | | | | |

| Composé A | | | |
|---|---|---|---|
| | Isohexadécane | Triglycéride C8C10 | Marcol™52 |
| 3h d'agitation | Pas de gélification | Pas de gélification | Pas de gélification |

On a ensuite tracé d'écoulement de solution d'huile comportant 5% massique de polymère et 2% d'eau au moyen d'un rhéomètre CARRIMED™ CLS 500 commercialisé par la société Texas Instruments pourvu d'un logiciel de traitement de données CLS. A partir de ces courbes d'écoulement, la viscosité à gradient nul de chacun des composés 2, 3 et 4 a été déterminée par extrapolation des données dans la zone du plateau newtonien.

Les résultats exprimés en Pas sont consignés dans le tableau suivant :

| | |
|---|---|
| Composé 2 | 5,26 Pa.s |
| Composé 3 | 4,39 Pa.s |
| Composé 4 | 13,36 Pa.s |

Ces essais mettent en évidence que les ionomères multiblocs selon l'invention, sont de bons agents épaississants d'huile en ce qu'ils modifient leur rhéologie en nécessitant le minimum d'eau, ce qui permet d'obtenir aisément des gels clairs, contrairement au polymère de l'état de la technique.

## Revendications

1. Copolymère ionomère multibloc **caractérisé en ce qu'**il comporte pour 100% molaire :
a) de 90% molaire à 99,9% molaire d'unités monomériques hydrophobes (A) issues d'un composé de formule (I) : 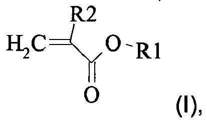 dans laquelle R1 représente un radical alkyle, linéaire ou ramifié, comportant de 12 à 22 atomes de carbone et R2 représente un atome d'hydrogène ou un radical méthyle ; et soit
b) de 0,1% molaire à 10% molaire d'unités monomériques (B) issue de l'acide 2-méthyl 2-[(1-oxo 2-propènyl) amino] 1-propanesulfonique (dénommé aussi acide 2-acrylamido 2-méthyl propanesulfonique) partiellement ou totalement salifiée, soit
c) de 0,1% molaire à 10% molaire d'unités monomériques (C) issues d'au moins un monomère cationique, soit
d) de 0,1% molaire à 10% molaire d'à la fois de dites unités monomériques (B) et de dites unités monomériques (C).

2. Copolymère ionomère multibloc tel que défini à la revendication 1, pour lequel, dans la formule (I), le radical R1 représente un radical alkyle linéaire choisi parmi les radicaux tétradécyle, hexadécyle, octadécyle, écosyle ou docosanyle.

3. Copolymère ionomère multibloc tel que défini à l'une des revendications 1 ou 2, pour lequel, ladite unité monomérique (C) optionnellement présente est issue d'un dérivé d'ammonium quaternaire choisis parmi les sels de N,N,N-triméthyl 2-[2-méthyl (1-oxo-2-propènyl) amino] propanammonium, les sels de N,N,N-triméthyl 2-[(1-oxo-2-propènyl) oxy] éthanammonium, les sels de N,N,N-triméthyl 3-[(1-oxo-2-propènyl) oxy] propanammonium, les sels de N,N,N-triméthyl 3-[(1-oxo-2-propènyl) amino] propanammonium, les sels de N,N,N-triméthyl 2-[(2-méthyl 1-oxo-2-propènyl) oxy] éthanammonium ou les sels de diallyl diméthyl ammonium.

4. Copolymère ionomère multibloc tel que défini à l'une des revendications 1 à 3, dans lequel les ratios molaires (A)/(B), (A)/(C) ou (A)/[(B)+(C)] sont inférieurs ou égaux à 99/1,

5. Copolymère ionomère multibloc tel que défini à la revendication 4, dans lequel les ratios molaires (A)/(B), (A)/(C) ou (A)/[(B)+(C)] sont inférieurs ou égaux à 98/2.

6. Copolymère ionomère multiblocs de méthacrylate de stéaryle et de 2-méthyl 2-[(1-oxo 2-propènyl) amino] 1-propanesulfonate de sodium pour le quel le ratio molaire (A)/(B) est supérieur ou égal à 95/5 et inférieur ou égal à 98/2.

7. Copolymère ionomère multiblocs de méthacrylate de stéaryle, de 2-méthyl 2-[(1-oxo 2-propènyl) amino] 1-propanesulfonate de sodium et de chlorure de N,N,N-triméthyl 2-[(2-méthyl 1-oxo-2-propènyl) oxy] éthanommonium, pour lequel le ratio molaire (A)/(B)/ (C) est compris entre 92/4/4 et 98/1/1.

8. Procédé de préparation du copolymère ionomère multibloc tel que défini à l'une des revendications 1 à 7, comprenant :
- Une étape a) de préparation d'une phase organique par mélange d'au moins une huile apolaire avec le monomère de formule (I) et un système tensioactif (T), susceptible de former des micelles inverses ;
- Le cas échéant, une étape b) d'addition dans la phase organique préparée à l'étape a), d'une solution aqueuse comprenant ledit monomère comportant la fonction acide sulfonique ;
- Le cas échéant, une étape b') d'addition dans le mélange préparé à l'étape a) ou dans le mélange préparé à l'étape b), d'une solution aqueuse comprenant ledit monomère cationique ;
- Une étape c) de polymérisation amorcée par introduction dans le mélange résultant de l'étape b) ou de l'étape b'), d'un amorceur thermique ; et
- Une étape d) d'élimination de l'huile et si désiré, dudit système tensioactif.

9. Variante du procédé tel que défini à la revendication 8, dans lequel l'étape d) est limitée à l'élimination de l'huile pour former un mélange dudit copolymère ionomère multibloc et dudit système tensioactif susceptible de former des micelles inverses.

10. Composition (F) comprenant pour 100% massique :
- De 60% à 90% massique, dudit copolymère ionomère multibloc tel que défini à l'une des revendications 1 à 8, et
- De 10% à 40% massique dudit système tensioactif susceptible de former des micelles inverses,
obtenue par la variante du procédé telle que définie à la revendication 9.

11. Utilisation du copolymère ionomère multibloc tel que défini à l'une des revendications 1 à 7 ou de la composition (F) telle que définie à la revendication 10, comme modificateur de rhéologie de la phase grasse d'une composition topique cosmétique, dermopharmaceutique ou pharmaceutique.

12. Utilisation telle que définie à la revendication 11, dans laquelle la composition topique cosmétique, dermopharmaceutique ou pharmaceutique est une émulsion de type eau dans huile.

13. Procédé pour modifier la rhéologie d'une composition topique cosmétique, dermopharmaceutique ou pharmaceutique comprenant une phase grasse, **caractérisé en ce que** l'on introduit dans ladite phase grasse une quantité efficace copolymère ionomère multibloc tel que défini à l'une des revendications 1 à 7 ou de la composition (F) telle que définie à la revendication 10.

## Patentansprüche

1. Mehrteiliges Copolymer-Ionomer, **dadurch gekennzeichnet, dass** es pro 100 Mol-% Folgendes aufweist:
a) 90 Mol-% bis 99,9 Mol-% hydrophobe Monomereinheiten (A), die aus einer Verbindung der folgenden Formel (I) hervorgegangen sind: 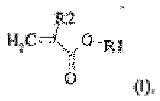 in der R1 ein lineares oder verzweigtes Alkylradikal mit 12 bis 22 Kohlenstoffatomen darstellt und R2 ein Wasserstoffatom oder ein Methylradikal darstellt, und entweder
b) 0,1 Mol-% bis 10 Mol-% Monomereinheiten (B), die aus 2-Methyl-2-[(1-oxo-2-propenyl)amino]-1-propansulfonsäure (auch als 2-Acrylamido-2-methylpropansulfonsäure bezeichnet), die teilweise oder vollständig in ein Salz überführt ist, hervorgegangen sind, oder
c) 0,1 Mol-% bis 10 Mol-% Monomereinheiten (C), die aus mindestens einem kationischen Monomer hervorgegangen sind, oder
d) 0,1 Mol-% bis 10 Mol-% der Monomereinheiten (B) und der Monomereinheiten (C) gleichzeitig.

2. Mehrteiliges Copolymer-Ionomer nach Anspruch 1, bei dem in der Formel (I) das Radikal 1 ein lineares Alkylradikal darstellt, das aus den Tetradecyl-, Hexadecyl-, Octadecyl-, Ecosyl- oder Docosanyl-Radikalen ausgewählt ist.

3. Mehrteiliges Copolymer-Ionomer nach einem der Ansprüche 1 oder 2, bei dem die optional vorhandene Monomereinheit (C) aus einem quaternären Ammoniumderivat hervorgegangen ist, das ausgewählt ist aus den N,N,N-Trimethyl 2-[2-methyl (1-oxo-2-propenyl)amino]propanammonium-Salzen, den N,N,N-Trimethyl 2-[(1-oxo 2-propenyl)oxy]ethanammonium-Salzen, den N,N,N-Trimethyl 3-[(1-oxo 2-propenyl)oxy]propanammonium-Salzen, den N,N,N-Trimethyl 3-[(1-oxo 2-propenyl)amino]propanammonium-Salzen, den N,N,N-Trimethyl 2-[(2-methyl 1-oxo-2-propenyl)oxy]ethanammonium-Salzen oder den Diallyldimethylammonium-Salzen;

4. Mehrteiliges Copolymer-Ionomer nach einem der Ansprüche 1 bis 3, bei dem die Molverhältnisse (A):(B), (A):(C) oder ((A):[(B)+(C)] kleiner oder gleich 99:1 sind.

5. Mehrteiliges Copolymer-Ionomer nach Anspruch 4, bei dem die Molverhältnisse (A):(B), (A):(C) oder ((A):[(B)+(C)] kleiner oder gleich 98:2 sind.

6. Mehrteiliges Copolymer-Ionomer aus Stearylmethacrylat und aus 2-Methyl-2-[(1-oxo-2-propenyl)amino]1-Natriumpropansulfonat, bei dem das Molverhältnis (A):(B) größer oder gleich 95:5 und kleiner oder gleich 98:2 ist.

7. Mehrteiliges Copolymer-Ionomer aus Stearylmethacrylat, aus 2-Methyl-2-[(1-oxo-2-propenyl)amino]1-Natriumpropansulfonat und aus N,N,N-Trimethyl-2-[(2-methyl 1-oxo-2-propenyl)oxy]ethanommonium-Chlorid, bei dem das Molverhältnis (A):(B):(C) zwischen 92:4:4 und 98:1:1 beträgt.

8. Verfahren zur Herstellung des mehrteiligen Copolymer-Ionomers nach einem der Ansprüche 1 bis 7, umfassend:
- einen Schritt a) der Herstellung einer organischen Phase durch Mischen mindestens eines apolaren Öls mit dem Monomer der Formel (I) und eines Tensidsystems (T), das inverse Mizellen bilden kann;
- gegebenenfalls einen Schritt b) der Zugabe einer wässrigen Lösung, die das Monomer enthält, das die Schwefelsäurefunktion aufweist, in die in Schritt a) hergestellte Phase;
- gegebenenfalls einen Schritt b') der Zugabe einer wässrigen Lösung, die das kationische Monomer enthält, in das in Schritt a) hergestellte Gemisch oder in das in Schritt b) hergestellte Gemisch;
- einen Schritt c) der Polymerisation, die durch Einbringen eines thermischen Initiators in das aus Schritt b) oder aus Schritt b') resultierende Gemisch initiiert wird; und
- einen Schritt d) des Entfernens des Öls, und, falls gewünscht, des Tensidsystems.

9. Variante des Verfahrens nach Anspruch 8, bei dem Schritt d) auf das Entfernen des Öls beschränkt ist, um ein Gemisch des mehrteiligen Copolymerlonomers und des Tensidsystems, das inverse Mizellen bilden kann, zu bilden.

10. Zusammensetzung (F), die pro 100 Masse-% Folgendes enthält:
- 60 Masse-% bis 90 Masse-% des mehrteiligen Copolymer-Ionomers nach einem der Ansprüche 1 bis 8 und
- 10 Masse-% bis 40 Masse-% des Tensidsystems, das inverse Mizellen bilden kann,
die durch die Variante des Verfahrens nach Anspruch 9 erhalten wird.

11. Verwendung des mehrteiligen Copolymer-Ionomers nach einem der Ansprüche 1 bis 7 oder der Zusammensetzung (F) nach Anspruch 10 als Rheologie-Modifizierer der Fettphasse einer kosmetischen, dermopharmazeutischen oder pharmazeutischen topischen Zusammensetzung.

12. Verwendung der Zusammensetzung nach Anspruch 11, wobei die kosmetische, dermopharmazeutische oder pharmazeutische topische Zusammensetzung eine Emulsion des Typs Wasser-in-Öl ist.

13. Verfahren zum Modifizieren der Rheologie einer kosmetischen, dermopharmazeutischen oder pharmazeutischen topischen Zusammensetzung, die eine Fettphase enthält, **dadurch gekennzeichnet, dass** in die Fettphase eine wirksame Menge des mehrteiligen Copolymer-Ionomers nach einem der Ansprüche 1 bis 7 oder der Zusammensetzung (F) nach Anspruch 10 eingebracht wird.

## Claims

1. Multiblock ionomer copolymer, **characterised in that** it comprises for 100 mol.%:
a) from 90 mol.% to 99.9 mol.% of hydrophobic monomer units (A) derived from a compound of formula (I): 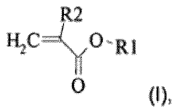 in which R1 represents a linear or branched alkyl radical comprising from 12 to 22 carbon atoms and R2 represents a hydrogen atom or a methyl radical; and any of
b) from 0.1 mol.% to 10 mol.% of monomer units (B) derived from partially or fully salified 2-methyl 2-[(1-oxo 2-propenyl) amino] 1-propanesulfonic acid (also known as 2-acrylamido 2-methyl propanesulfonic acid), or
c) from 0.1 mol.% to 10 mol.% of monomer units (C) derived from at least one cationic monomer, or
d) from 0.1 mol.% to 10 mol.% of both said monomer units (B) and said monomer units (C).

2. Multiblock ionomer copolymer according to claim 1, wherein, in formula (I), the radical R1 represents a linear alkyl radical selected from among tetradecyl, hexadecyl, octadecyl, ecosyl and docosanyl radicals.

3. Multiblock ionomer copolymer according to either claim 1 or claim 2, wherein said optional monomer unit (C) is derived from a quaternary ammonium derivative selected from among the salts of N,N,N-trimethyl 2-[2-methyl (1-oxo-2-propenyl) amino] propanammonium, the salts of N,N,N-trimethyl 2-[(1-oxo-2-propenyl) oxy] ethanammonium, the salts of N,N,N-trimethyl 3-[(1-oxo-2-propenyl) oxy] propanammonium, the salts of N,N,N-trimethyl 3-[(1-oxo-2-propenyl) amino] propanammonium, the salts of N,N,N-trimethyl 2-[(2-methyl 1-oxo-2-propenyl) oxy] ethanammonium or the salts of diallyl dimethyl ammonium.

4. Multiblock ionomer copolymer according to any of claims 1 to 3, wherein the molar ratios (A)/(B), (A)/(C) or (A)/[(B)+(C)] are less than or equal to 99/1.

5. Multiblock ionomer copolymer according to claim 4, wherein the molar ratios (A)/(B), (A)/(C) or (A)/[(B)+(C)] are less than or equal to 98/2.

6. Multiblock ionomer copolymer of stearyl methacrylate and of 2-methyl 2-[(1-oxo 2-propenyl) amino 1-sodium propanesulfonate, wherein the molar ratio (A)/(B) is greater than or equal to 95/5 and less than or equal to 98/2.

7. Multiblock ionomer copolymer of stearyl methacrylate and of 2-methyl 2-[(1-oxo 2-propenyl) amino] 1-sodium propanesulfonate and of N,N,N-trimethyl 2-[(2-methyl 1-oxo-2-propenyl) oxy] ethanammonium chloride, wherein the molar ratio (A)/(B)/(C) is between 92/4/4/ and 98/1/1.

8. Method for preparing the multiblock ionomer copolymer according to any of claims 1 to 7, comprising:
- a step a) of preparing an organic phase by mixing at least one apolar oil with the monomer of formula (I) and a surface-active agent (T) capable of forming reverse micelles;
- where necessary, a step b) of adding an aqueous solution comprising said monomer having the sulfonic acid function to the organic phase prepared in step a);
- where necessary, a step b') of adding an aqueous solution comprising said cationic monomer to the mixture prepared in step a) or to the mixture prepared in step b);
- a step c) of polymerisation initiated by the introduction of a thermal initiator to the mixture produced in step b) or step b'); and
- a step d) of eliminating the oil and, if required, said surface-active agent.

9. Variant of the method according to claim 8, wherein step d) is limited to eliminating the oil to form a mixture of said multiblock ionomer copolymer and said surface-active agent capable of forming reverse micelles.

10. Composition (F) comprising for 100 % by weight:
- from 60 % to 90 % by weight of said multiblock ionomer copolymer according to any of claims 1 to 8, and
- from 10 % to 40 % by weight of said surface-active agent capable of forming reverse micelles,
obtained by the variant of the method according to claim 9.

11. Use of the monoblock ionomer copolymer according to any of claims 1 to 7 or of the composition (F) according to claim 10 as a rheology modifier of the oil phase of a cosmetic, dermopharmaceutical or pharmaceutical topical composition.

12. Use according to claim 11, wherein the cosmetic, dermopharmaceutical or pharmaceutical topical composition is a water-in-oil emulsion.

13. Method for modifying the rheology of a cosmetic, dermopharmaceutical or pharmaceutical topical composition comprising an oil phase, **characterised in that** an effective amount of multiblock ionomer copolymer according to any of claims 1 to 7 or of the composition (F) according to claim 10 is introduced to said oil phase.
